# EUROPEAN PATENT APPLICATION

(11) **EP 4 141 120 A1**
(43) Date of publication of application: **01.03.2023**
(21) Application number: 21791771.5
(22) Date of filing: 22.04.2021
(51) Int. Cl.: C12N 15/82, C07K 14/005

(54) **CORONAVIRUS DISEASE 2019 (COVID-19) RECOMBINANT SPIKE PROTEIN FORMING TRIMER, METHOD FOR MASS PRODUCING RECOMBINANT SPIKE PROTEIN IN PLANTS, AND METHOD FOR PREPARING VACCINE COMPOSITION ON BASIS THEREOF**

(30) Priority: 22.04.2020 KR 20200048980
(71) Applicant: POSTECH Research and Business Development Foundation, Gyeongsangbuk-do 37673 (KR); Korea National Institute of Health, Cheongju-si, Chungcheongbuk-do 28159 (KR); Bioapplications Inc., Pohang-si, Gyeongsangbuk-do 37668 (KR)
(72) Inventor: HWANG, In, Hwan, Pohang-si, Gyeongsangbuk-do 37673 (KR); SONG, Shijian, Pohang-si, Gyeongsangbuk-do 37673 (KR); MD, Rezaul, Islam, Khan, Pohang-si, Gyeongsangbuk-do 37673 (KR); DIAO, Haiping, Pohang-si, Gyeongsangbuk-do 37673 (KR); SOHN, Eun, Ju, Pohang-si, Gyeongsangbuk-do 37668 (KR); CHOI, Bo, Hwa, Pohang-si, Gyeongsangbuk-do 37668 (KR); CHOI, Jang, Hoon, Cheongju-si, Chungcheongbuk-do 28159 (KR); JANG, Eun, Young, Cheongju-si, Chungcheongbuk-do 28159 (KR); LEE, Young, Jae, Cheongju-si, Chungcheongbuk-do 28159 (KR)
(74) Representative: Dehns
(86) International application number: PCT/KR2021/005124
(87) International publication number: WO 2021/215857

(57) **Abstract**

The present invention relates to a recombinant spike protein of the COVID-19 virus forming a trimer and a method for mass-producing the recombinant spike protein, and more specifically to a method for designing a recombinant gene expressing a recombinant spike protein of the COVID-19 virus forming a trimer for the purposes of enhancing immunogenicity and effective antigen delivery, and a method for mass-producing the recombinant spike protein in plants.

## Description

### CROSS-REFERENCE TO RELATED APPLICATION

This application claims priority to and the benefit of Korean Patent Application No. 10-2020-0048980, filed on April 22, 2020, the disclosure of which is incorporated herein by reference in its entirety.

### [Technical Field]

The present invention relates to a recombinant spike protein of the COVID-19 virus forming a trimer and a method for mass-producing the recombinant spike protein, and more specifically to a method for designing a recombinant gene expressing a recombinant spike protein of the COVID-19 virus forming a trimer for the purposes of enhancing immunogenicity and effective antigen delivery, and a method for mass-producing the recombinant spike protein in plants. In addition, the present invention provides an effective vaccine material against COVID-19 by using a trimeric spike protein produced in plants.

### [Background Art]

Recently, the possibility of low-cost production of recombinant proteins in plants has been proposed, and various attempts have been made therefrom (Schillberg *et al.,* 2003; Holtz *et al.,* 2015; Marusic *et al.,* 2016). In particular, studies are being conducted to confirm the production potential of various medical proteins and the like. The production of recombinant proteins in plants can have various advantages, one of which is that there are almost no toxins such as endotoxins present in microorganisms such as *E. coli,* and there are no pathogens that can infect the human body. In addition, it is known that there are no harmful proteins such as prions, and thus, it is possible to produce recombinant proteins that are safer than animal cells or microorganisms. In addition, it is much cheaper than animal cells in terms of manufacturing cost, and it is more economical than microorganisms such as *E*. *coli* in large-scale production according to the method of cultivating plants. In order to realize this possibility, it is necessary to develop several essential technologies. Among them, the first and most important technology is the development of an expression vector capable of inducing high gene expression in plants (Staub *et al.,* 2000; Regnard *et al.,* 2010). In plants, gene expression can be induced through various methods. Various methods are possible, such as a method of integrating a recombinant gene into the genome of a plant, a method of integrating the genome of a chloroplast, and a method of transiently expressing a gene using *Agrobacterium* (Arzola *et al.,* 2011; Werner *et al.,* 2011). The method of integrating recombinant genes into nuclear genome or chloroplast genome basically produces proteins in plants through the process of securing transformants. On the other hand, when protein is produced by inducing the transient expression of genes by infiltrating *Agrobacterium* into plant tissue, the production process of transformants is not included, and thus, the protein production period is short, and in general, compared to protein production through transformants, it has the advantage of a remarkably high level of production (Arzola *et al.,* 2011). In addition, since the expression suppression mechanism of other genes in plants can be suppressed by co-infiltration of gene silencing suppressors, it is possible to induce higher protein expression levels (Garabagi *et al.,* 2011). However, whenever transient expression is desired, there are disadvantages that the *Agrobacterium* culture introduced with a binary vector including the target gene and the *Agrobacterium* culture introduced with a binary vector expressing the p38 gene silencing suppressor must be separately prepared and mixed at an appropriate ratio to perform the process of co-infiltration. In particular, in the case of culturing two types of *Agrobacterium,* there are limitations in terms of time and economic feasibility.

Coronavirus is one of the three major viruses that cause common colds in humans along with adenovirus and rhinovirus, and it is an RNA virus with a gene size of 27 to 32 kb that can variously infect humans. When viewed with an electron microscope, the surface of the virus particle protrudes like a protrusion, and this shape resembles a crown, and thus, it was named after the Latin word "corona" meaning crown. It accounts for 10 to 30% of adult colds that occur mainly in the cold winter, and the main symptom is a nasal cold accompanied by a headache, sore throat or cough. Since the coronavirus was first discovered in chickens in the 1930s, it has been found in animals such as dogs, pigs and birds, and in humans in the 1960s. Coronavirus has been found in both animals and humans, and as the area of human activity expands, the virus that was prevalent only among animals causes genetic mutations in order to survive and is passed on to humans. Examples include SARS (bats and civets), MERS (bats and camels) and COVID-19 (probably bats). The coronaviruses discovered so far are classified into four genera: alpha, beta, gamma and delta. Herein, alpha is further divided into types 1a and 1b, and beta is divided into types 2a, 2b, 2c and 2d. Of these, alpha and beta infect humans and animals, and gamma and delta infect animals. There are a total of 7 types of human-infecting coronaviruses that have been identified so far, including HCoV 229E, HCoV NL63, HCoV OC43, HCoV HKU1, SARS-CoV, MERS-CoV and SARS-CoV-2. Of these, four types (229E, OC43, NL63, HKU1) cause only mild symptoms similar to common colds. However, SARS (severe acute respiratory syndrome), MERS (MERS-CoV) and COVID-19 (SARS-CoV-2, severe acute respiratory syndrome coronavirus 2) may cause serious respiratory disease such as severe pneumonia and result in many deaths.

COVID-19 virus is a new type of coronavirus (SARS-CoV-2) that first emerged in Wuhan, China in December 2019 and has spread throughout China and around the world. The COVID-19 virus has a very high transmission rate, making it particularly contagious. After being infected with the COVID-19 virus, after an incubation period of about 2 to 14 days (estimated), the main symptoms include fever (37.5 degrees), respiratory symptoms such as cough or shortness of breath and pneumonia, but asymptomatic infections are not uncommon. The spike (S) protein of the COVID-19 virus (COVID-19) is a type 1 membrane glycoprotein containing a very large ectodomain region, a single transmembrane domain (TMD) and a short cytoplasmic tail. The spike protein, like the spike (S) protein of other coronaviruses, exists as a trimer on the surface of the virus, has a receptor binding domain required for the virus to invade the host cell and a fusion peptide that induces fusion between the virus membrane and organelle membrane during cell invasion, and is known to play a role, such as inducing neutralized antibodies against the spike protein in a natural host. The corona spike protein exists as a trimer on the surface of the virus membrane. Therefore, the trimeric type spike protein is thought to act as an antigen, and the trimer is thought to be important for the induction of neutralizing antibodies. Similarly, in the case of influenza virus, the HA protein exists as a trimer on the surface of the virus, and it is known that the formation of this trimer has high antigenicity.

Recombinant proteins are excellent in safety, but have low immunogenicity and high production cost compared to live viruses. Therefore, it is essential to produce a highly immunogenic recombinant protein vaccine capable of inducing various immune responses and inducing a high immune response for efficient prevention by using this highly safe recombinant protein, and there is also a need for recombinant proteins engineered to enable effective delivery.

It was attempted to develop a vaccine by using the recombinant spike protein of the COVID-19 virus. The spike protein is processed into two subunits S 1 and S2 during COVID-19 infection, and the domain binding to the receptor required for infection exists in S 1, and S2 has a fusion peptide. Therefore, it is not easy to predict what effect it will have on the protective immune effect when S1, S2 or full-length antigen is used. Since S1 has RBD, it will be able to induce an antibody that binds to RBD, and through the binding of this antibody, it will interfere with the binding to the receptor, thereby preventing virus infection. On the other hand, since S 1 includes the part where the most mutations are introduced, there is a possibility that it cannot cover various variants. On the other hand, in the case of S2, it can be predicted that the induced antibodies will not be able to prevent the virus from binding to the receptor, but may generate antibodies that interfere with the fusion step. In the case of full length, since both parts are included, it may be possible to induce an antibody that interferes with the binding of the virus to the receptor and an antibody that interferes with the fusion process. However, it may be more difficult compared to S 1 and S2 in the production of proteins used as antigens.

Based on these various considerations, it was attempted to construct a recombinant gene for producing a spike protein as an antigen in plants. For mass production of recombinant proteins, it was attempted to make recombinant proteins using the full-length ectodomain excluding TMD and the cytosolic domain, and in particular, it was attempted to increase antigenicity by making a trimer using only the ectodomain, which is the form where the spike protein exists on the surface of the virus. To this end, a fragment (named as SfΔ(TMD-CT)) including a total of 1,198 residues from the 16^{th} amino acid to the 1,213^{th} amino acid in the spike ((s)) of the COVID-19 virus was used. In addition, by using the S1 subunit (named S1s) including from the N-terminus to the 16^{th} amino acid to the 681^{st} amino acid of sub-domain 2 (SD2) without a leader sequence in the spike protein of the coronavirus; and a fragment without TMD and the cytosolic tail domain present in the C-terminal region of the S2 subunit, which is known to be less mutated among spike proteins (a region including from the 682^{nd} amino acid to the 1,213^{th} amino acid, a total of 532 residues, named as [S2sΔ (TMD-CT)]), it was attempted to construct an expression system in plants. These two types of recombinant proteins produced in plants were made, and they were used as antigens to develop vaccines. The full length of the spike protein forms a trimer when present on the surface of the virus, but among the genes encoding the spike protein, if recombinant proteins were prepared using an ectodomain without TMD and the cytosolic region or a portion encoding S2 without TMD and the cytosolic domain, it was expected that the recombinant proteins would not form a trimer well. It was attempted to develop a technique for producing these two types of recombinant proteins in the form of trimers in plants for use in vaccines. A binary vector was constructed that allows high expression of the vector capable of expressing the recombinant spike proteins constructed in this way in plants.

In addition, the present invention is directed to providing a vaccine composition by pure isolation and purification of these proteins made in plant cells. In order to increase the yield of protein produced in plant cells, it is important to have an appropriate buffer composition. Since these proteins are large in size and are heavily glycosylated proteins, it is necessary to compare and analyze various buffer compositions for solubilization to secure the optimal buffer composition. In addition, the isolation and purification of proteins were attempted by pure isolation and purification through Ni²⁺-NTA affinity column chromatography and size exclusion gel filtration column chromatography by using a His tag present in the C-terminus.

In addition, by using various proteins in the trimeric form of the spike protein produced in this way, the degree of antibody induction can be confirmed through immunization in mice and hamsters, and the degree of protection (PRNT50) of the induced antibodies can be confirmed. In addition, in the case of hamsters, since they have a certain degree of sensitivity to COVID-19, it is possible to confirm the degree of suppression of virus proliferation through the actual challenge inoculation of COVID-19. Through this process, optimal vaccine candidates can be selected.

### [Disclosure]

### [Technical Problem]

The present invention has been devised to solve the above problems, and an object of the present invention is to provide a recombinant vector for producing a recombinant spike protein of a coronavirus forming a trimer SfΔ(TMD-CT):mCor1:Hisx5:HDEL (named S_{full-t}), S1:mCor1:Hisx5:HDEL (named S1s-t) or S2Δ(TMD-CT):mCor1:Hisx5:HDEL (named S2s-t), including (i) a gene encoding a protein (SfΔ(TMD-CT)) lacking an amino acid sequence from the transmembrane domain to the C-terminus of the full-length spike protein of a coronavirus; an S1 subunit protein (S1s) including an amino acid sequence from the N-terminus to sub-domain 2 (SD2) excluding a leader sequence in the spike protein of a coronavirus; or a protein lacking an amino acid sequence from the transmembrane domain in subunit 2 to the C-terminus of the spike protein of a coronavirus; and (ii) a gene encoding a protein of a trimeric motif region of mouse Coronin 1 (mCor1); a gene encoding five His residues for isolation and purification; and a gene encoding HDEL, which is an ER retention motif for accumulation in the endoplasmic reticulum of plants. In addition, it is directed to providing a recombinant vector for producing SfΔ(TMD-CT):Hisx5:HDEL protein (named S_{full-m}), S1:Hisx5:HDEL protein (named S1s-m) or S2Δ(TMD-CT):HisxS:HDEL protein (named S2s-m) without mCor as control groups

Another object of the present invention is to provide a method for producing a recombinant spike protein of a coronavirus forming a trimer in a plant, including the steps of:
(a) constructing the aforementioned recombinant vector;
(b) preparing a transgenic organism by introducing the recombinant vector into an organism;
(c) culturing the transgenic organism;
(d) infiltrating the culture product into a plant; and
(e) pulverizing the plant to obtain a recombinant spike protein of a coronavirus forming a trimer.

Another object of the present invention is to provide a condition for isolation and purification from a plant extract with high efficiency, after expressing SfΔ(TMD-CT):mCor1:Hisx5:HDEL (named S_{full-t}), S1s:mCor1:Hisx5:HDEL (named S1s-t) or S2sΔ(TMD-CT):mCor1:Hisx5:HDEL (named S2s-t), which are spike protein-derived recombinant proteins, in plant cells.

Another object of the present invention is to provide a vaccine composition that effectively treats COVID-19 by using two types of SfΔ(TMD-CT):mCor1:Hisx5:HDEL and S2sA(TMD-CT):mCor1:Hisx5:HDEL, among these several spike-derived recombinant proteins.

### [Technical Solution]

The present invention provides a recombinant vector for producing a recombinant spike protein S_{full}-t or S2s-t of SARS-CoV-2 forming a trimer, including (i) a gene including a protein SfΔ(TMD-CT) lacking an amino acid sequence from the transmembrane domain to the C-terminus of the spike protein of a coronavirus; a protein (S1s) including the S1 subunit of the spike protein of a coronavirus; or a protein lacking an amino acid sequence from TMD in subunit 2 to the C-terminus of the spike protein of a coronavirus; and (ii) a gene encoding a protein of a trimeric motif region of Coronin 1 (mCor1); a gene encoding five His residues for isolation and purification; and a gene encoding HDEL, which is an ER retention motif for accumulation in the endoplasmic reticulum of plants.

The coronavirus may be any one selected from the group consisting of SARS-CoV (SARS-Coronavirus), MERS-CoV (MERS-Coronavirus) and SARS-CoV-2 (SARS-Coronavirus-2).

The protein lacking an amino acid sequence from the transmembrane domain to the C-terminus of the spike protein of SARS-CoV-2 may include the amino acid sequence of SEQ ID NO: 2.

The gene encoding a protein lacking an amino acid sequence from the transmembrane domain to the C-terminus of the spike protein of SARS-CoV-2 may include the nucleotide sequence of SEQ ID NO: 1.

The protein (S1s) including the 16^{th} amino acid to the 681^{st} amino acid in S1 subunit of the spike protein of SARS-CoV-2 may include the amino acid sequence of SEQ ID NO: 4.

The gene encoding a protein including the 16^{th} amino acid to the 681^{st} amino acid in S1 subunit of the spike protein of SARS-CoV-2 may include the nucleotide sequence of SEQ ID NO: 3.

The protein including the 682^{nd} amino acid to the 1,213^{rd} amino acid in S2 subunit of the spike protein of SARS-CoV-2 may include the amino acid sequence of SEQ ID NO: 6.

The gene encoding a protein including the 682^{nd} amino acid to the 1,213^{rd} amino acid in S2 subunit of the spike protein of SARS-CoV-2 may include the nucleotide sequence of SEQ ID NO: 5.

The protein of a trimeric motif region of Coronin 1 (mCor1) may include the amino acid sequence of SEQ ID NO: 8.

The gene encoding a protein of a trimeric motif region of Coronin 1 (mCor1) may include the nucleotide sequence of SEQ ID NO: 7.

The gene encoding 5'-UTR may include the nucleotide sequence of SEQ ID NO: 14.

In addition, the present invention may provide a recombinant vector for producing a recombinant spike protein of a coronavirus forming a trimer, further including a gene encoding the protein of a Hisx5 tag in the recombinant vector.

In addition, the present invention may provide a recombinant vector for producing a recombinant spike protein of a coronavirus forming a trimer, further including a gene encoding the protein of the HDEL motif in the recombinant vector.

The protein of the HDEL motif may include the amino acid sequence of SEQ ID NO: 13.

The gene encoding the protein of the HDEL motif domain may include the nucleotide sequence of SEQ ID NO: 12.

The recombinant vector may further include any one promoter selected from the group consisting of a 35S promoter derived from cauliflower mosaic virus, a 19S RNA promoter derived from cauliflower mosaic virus, a Mac promoter, an actin protein promoter and ubiquitin protein promoter of a plant.

In addition, the present invention may provide a transgenic organism which is transformed by the recombinant vector.

The transgenic organism which is transformed may be a prokaryote or a eukaryote.

The present invention may provide a method for producing a recombinant spike protein of a coronavirus forming a trimer in a plant, including the steps of:
(a) constructing the aforementioned recombinant vector;
(b) preparing a transgenic organism by introducing the recombinant vector into an organism;
(c) culturing the transgenic organism;
(d) infiltrating the culture product into a plant; and
(e) pulverizing the plant to obtain a recombinant spike protein of a coronavirus forming a trimer.

The present invention may provide a method for producing a vaccine candidate material through a process of isolating and purifying S_{full-t} and S2s-t proteins from a plant extract by using Ni²⁺-NTA affinity column chromatography and size exclusion column chromatography.

Another object of the present invention is to provide a vaccine composition for effectively protecting against COVID-19 using these two types of spike proteins, S_{full-t} and S2s-t. These proteins may provide a vaccine composition alone with or without adjuvant, and the amount of protein may range from 1 microgram to 30 micrograms.

### [Advantageous Effects]

The recombinant spike protein of the COVID-19 virus forming a trimer according to the present invention constitutes a recombinant protein which has a gene encoding a Se protein consisting of an ectodomain (from the 16^{th} amino acid to the 1,213^{th} amino acid, a total of 1,198 residues) of the spike protein lacking from the transmembrane domain to the C-terminus of the spike protein of the COVID-19 virus, or a protein (a total of 666 residues) including from the 16^{th} amino acid to the 681^{st} amino acid of the S1 subunit of the spike protein of the coronavirus, or a protein (a total of 532 residues) including from the 682^{nd} amino acid to the 1,213^{th} amino acid of the S2 subunit lacking from the transmembrane domain to the C-terminus of the spike protein of the coronavirus, and a trimeric motif of mouse Coronin 1 to form a trimer to increase immunity, and includes HDEL at the C-terminal end to allow proteins to be accumulated in the endoplasmic reticulum of the plant, and thus can be prepared in large amounts in the plant, and includes a His tag domain at the C-terminal region to facilitate isolation and purification of the protein.

It includes a vaccine composition composed of S_{full-t}, or S2s-t recombinant protein of the S protein of COVID-19 produced in this way in plants. The vaccine composition may consist only of plant-produced proteins or may include an adjuvant such as alum.

### [Description of Drawings]

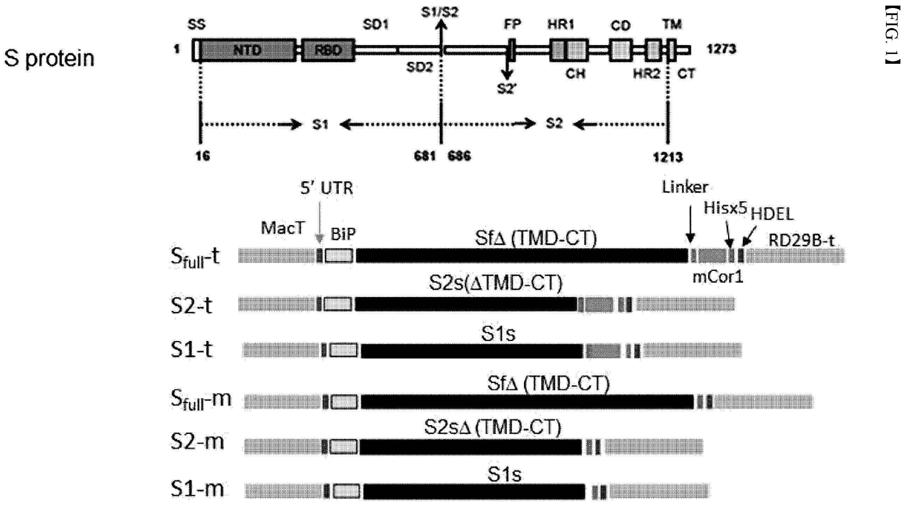

FIG. 1 is a diagram showing the construction of several recombinant vectors used in the present invention.

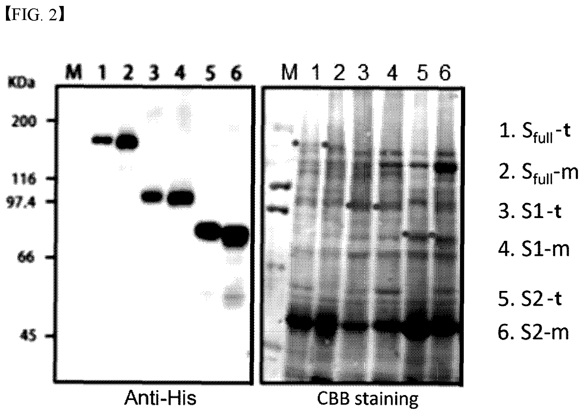

FIG. 2 shows the results of Western blot analysis using an anti-His antibody and staining with Coomassie brilliant blue after isolating by SDS/PAGE the total extract of *N. benthamiana,* which expressed SfΔ (TMD-CT), S1s and S2sΔ (TMD-CT) proteins having a His tag and HDEL, and these three types of proteins additionally expressed mCor1 protein.

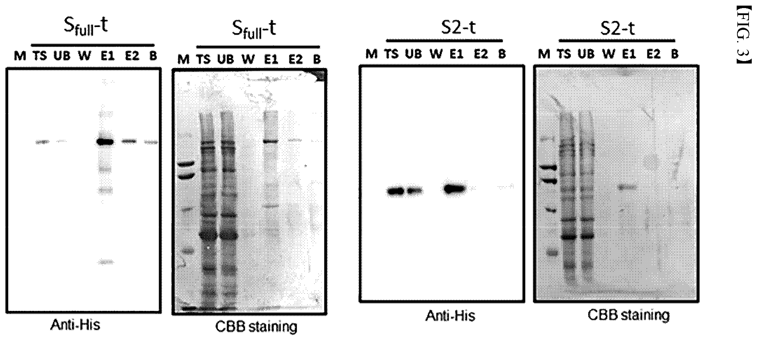

FIG. 3 shows the results of pure isolation and purification of S_{full-t} and S2s-t from *N. benthamiana* extract using Ni²⁺-NTA affinity column chromatography, development by SDS/PAGE, Western blot analysis using an anti-His antibody, and staining of the membrane with Coomassie brilliant blue.

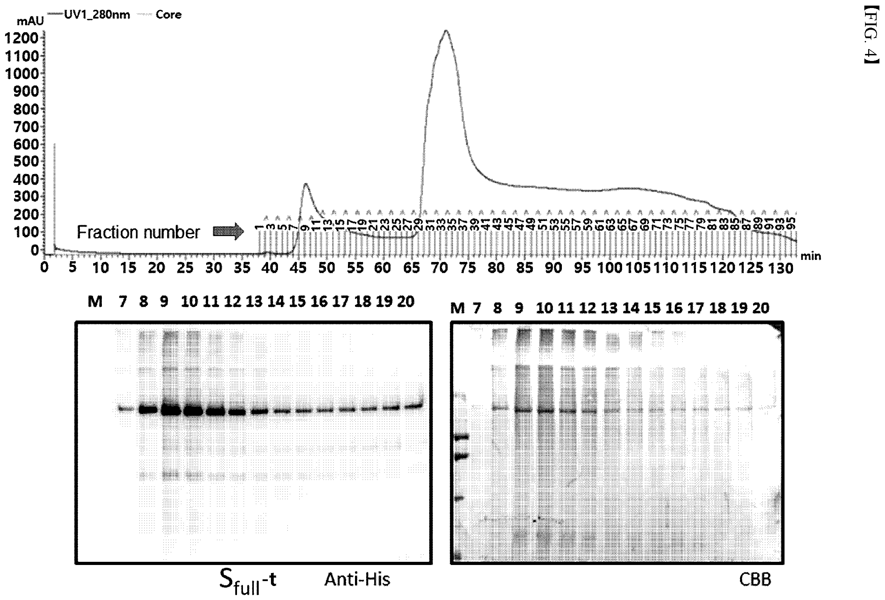

FIG. 4 shows the results of fractionation of S_{full-t} isolated from *N. benthamiana* extract using Ni²⁺-NTA affinity column chromatography by size-exclusion column chromatography, development by SDS/PAGE, and staining with Coomassie brilliant blue.

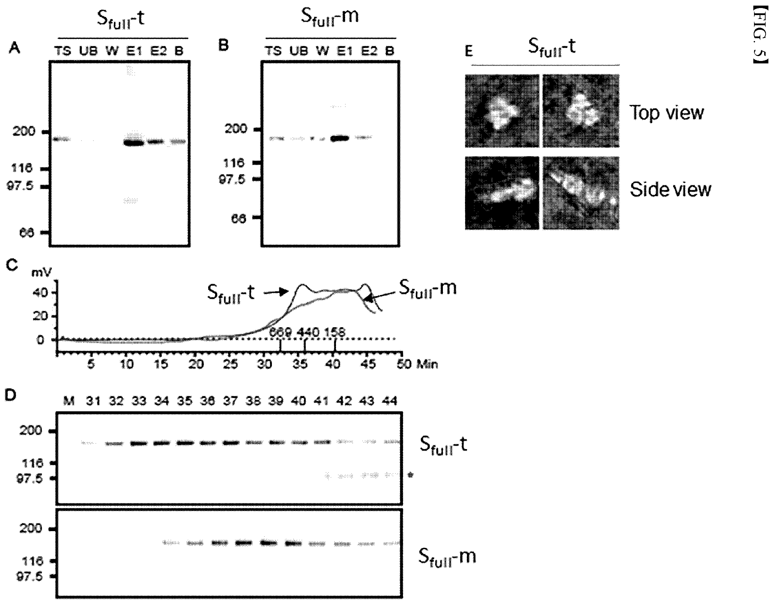

FIG. 5 shows the results of Western blot analysis using an anti-His antibody after pure isolation and purification of S_{full-t} and S_{full-m} isolated from *N. benthamiana* extract using Ni²⁺-NTA affinity column chromatography, fractionation of S_{full-t} and S_{full-m} by size exclusion column chromatography, and negative staining of the isolated and purified S_{full-t}, and confirmation of the morphology of the protein using an electron microscope, confirming that a trimer was formed.

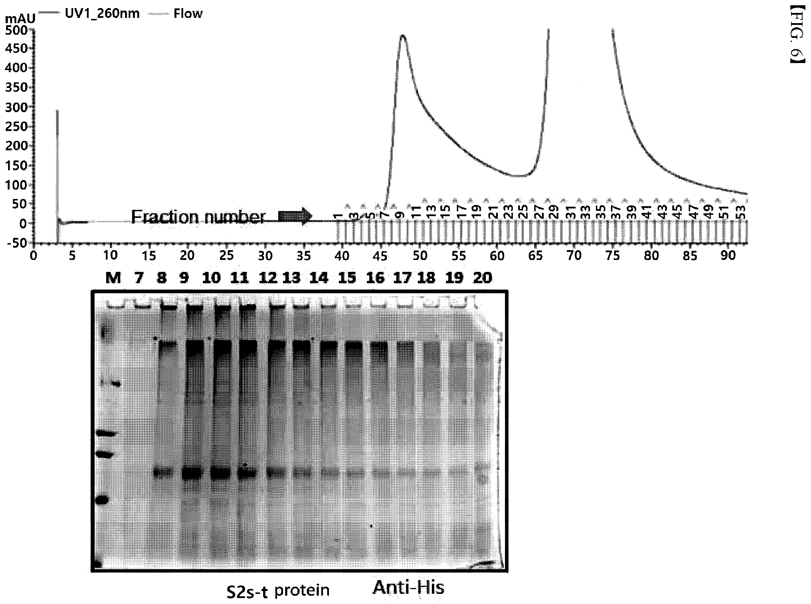

FIG. 6 shows the results of fractionation of S2s-t isolated from the *N. benthamiana* extract using Ni²⁺-NTA affinity column chromatography by size-exclusion column chromatography, development by SDS/PAGE and staining with Coomassie brilliant blue.

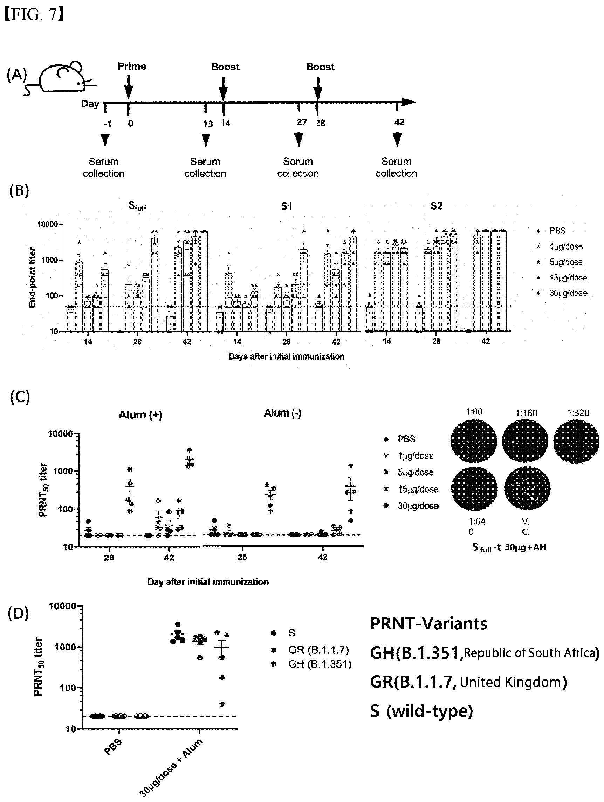

FIG. 7 shows the results of evaluation of the immunogenicity of COVID-19 plant vaccine candidate materials in experimental animals (mouse, Balb/c). For this, the results were shown after performing a humoral immune response analysis (FIG. 7B), a neutralizing antibody induction analysis (FIGS. 7C and 7D), and a cell-mediated immune response analysis (FIG. 7E).

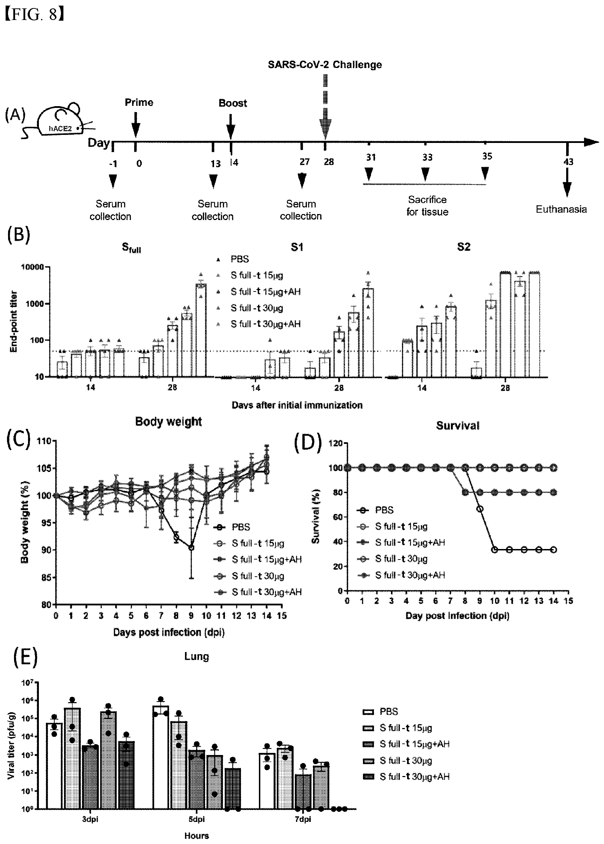

FIG. 8 shows the results of evaluation of the protective efficacy of the COVID-19 plant vaccine candidate materials in experimental animals (TG mouse), in which each immune antigen was immunized twice in total at an interval of 2 weeks by an intramuscular route, and in order to confirm the production of antibodies against the immune antigen, the results of evaluation of the immunogenicity such as the production of antibodies (IgGs) and the production of neutralizing antibodies in serum by performing blood collection before immunization, after primary immunization, and after 2 weeks of secondary immunization were shown (FIG. 8A), and the results of analysis of antibody induction according to plant vaccine immunization (FIG. 8B), and the results of analysis of the survival rate and tissue titer according to hamster challenge inoculation according to plant vaccine immunization (FIGS. 8C, 8D and 8E) were shown.

### [Best Mode]

Hereinafter, the present invention will be described in detail.

As a membrane protein of the COVID-19 virus, the spike protein, which is known to be important for infiltrating cells, is considered as a vaccine candidate that can prevent infection of the COVID-19 virus. In the present invention, various recombinant proteins were made based on the spike protein, and they were used to develop vaccines. The full length of the spike protein forms a trimer when present on the surface of the virus, but when a recombinant protein is made using a portion encoding only the ectodomain of all or part of the spike gene, it was expected that the recombinant protein will not form a trimer well. Therefore, in order to utilize these recombinant proteins as vaccines, it was thought that it was important to form a trimer as when the protein was present on the surface of the virus, and it was attempted to develop a technique of maintaining a trimer when these exist in a soluble form. In the present invention, when the recombinant protein of the entire ectodomain of the spike protein [SfΔ(TMD-CT)], S1s or the S2 region without TMD and C-terminus [S2sΔ(TMD-CT)] was expressed and produced in a plant, it was attempted to develop a technique for inducing the formation of a trimer. Accordingly, the ectodomain of the spike protein lacking from the spike transmembrane domain to the C-terminus of the COVID-19 virus that can be mass-produced (Se; a total of 1,198 residues from the 16^{th} amino acid to the 1,213^{th} amino acid), a site including from the N-terminus to the SD2 domain (S1s; a total of 666 residues from the 16^{th} amino acid to the 681^{st} amino acid), or a site without TMD and the C-terminus in the S2 subunit (S2sΔ(TMD-CT); a total of 532 residues from the 682^{nd} amino acid to the 1,213^{th} amino acid ) was fused to the trimeric motif of mouse Coronin 1 forming a trimeric structure, and additionally, a construct without mCor1 was constructed as a control group for the recombinant protein. A binary vector capable of the high expression of recombinant genes including parts of the above various spike proteins in plants was constructed.

The present invention may prove a recombinant vector for producing a recombinant spike protein of a coronavirus forming a trimer, including (i) a gene encoding a protein (SfA(TMD-CT)) lacking an amino acid sequence from the transmembrane domain to the C-terminus in the spike protein of a coronavirus; an S 1 subunit protein (S1s) including the N-terminus to sub-domain 2 (SD2) in the spike protein of a coronavirus; or a protein (S2sA(TMD-CT)) lacking an amino acid sequence from the transmembrane domain to the C-terminus in an S2 subunit of the spike protein of a coronavirus spike; and (ii) a gene encoding a protein of the trimeric motif region of Coronin 1 (mCor 1).

In order to develop a vaccine by using the recombinant spike protein of the COVID-19 virus, the ectodomain of the spike protein with the transmembrane domain to the C-terminus removed (full length ectodomain of S; from the 16^{th} amino acid to the 1213^{th} amino acid, a total of 1,198 residues), or a region from the N-terminus to sub domain 2 (SD2) (from the 16^{th} amino acid to the 681^{st} amino acid, a total of 666 residues), or the ectodomain of S2 (the 682^{nd} amino acid to the 1,213^{th} amino acid, a total of 532 residues) was used to create a recombinant protein, and it was used to develop a vaccine. The full length of the spike protein forms a trimer when present on the surface of the virus, but it was expected that a trimer will not be formed well if the recombinant protein is expressed using the ectodomain part of all or part of the gene encoding the spike protein, and it was expected to have low immunogenicity due to the non-formation of the trimer. Therefore, in the present invention, it was originally intended to make a recombinant protein of the entire trimeric form of the spike protein as the full-length spike protein exists on the surface of the virus, or the S1c ectodomain from the N-terminus to the SD2 domain, or the S2 ectodomain. To this end, in the present invention, COVID-19 was selected and the 32 residues from the 122^{nd} amino acid to the 153^{rd} amino acid of mouse Coronin1 (mCor1) were fused by using a linker having 12 amino acid residues to construct a construct.

Subsequently, a His tag with five His residues was fused for isolation and purification of the recombinant protein, and finally a HDEL motif was fused to accumulate in the ER to complete the construct (SfΔ(TMD-CT):mCor1:Hisx5:HDEL, S1s:mCor1:Hisx5:HDEL, S2sΔ(TMD-CT):mCor1:Hisx5:HDEL, FIG. 1). In addition, constructs without mCor1 were constructed from the above three types of the spike recombinant proteins and used as control groups.

The coronavirus may be any one selected from the group consisting of SARS-CoV (SARS-Coronavirus), MERS-CoV (MERS-Coronavirus) and SARS-CoV-2 (SARS-Coronavirus-2).

In the spike protein of the COVID-19 virus, the protein lacking from the transmembrane domain to the C-terminus may include the amino acid sequence of SEQ ID NO: 2.

The gene encoding the protein lacking from the transmembrane domain to the C-terminus in the spike protein of the COVID-19 virus may include the nucleotide sequence of SEQ ID NO: 1, and specifically, the gene may include a nucleotide sequence having a sequence homology of 70% or more, more preferably, 80% or more, still more preferably, 90% or more, and most preferably, 95% or more to the nucleotide sequence of SEQ ID NO: 1. The "% of sequence homology" to a polynucleotide is determined by comparing two optimally aligned sequences with a comparison region, and a portion of the polynucleotide sequence in the comparison region may include additions or deletions (*i.e*., gaps) compared to a reference sequence (not including additions or deletions) to the optimal alignment of the two sequences.

The S1s protein including from the N-terminus to sub-domain 2 (SD2) in the spike protein of the COVID-19 virus may include the amino acid sequence of SEQ ID NO: 4.

The gene encoding the S1s protein including the N-terminus to sub-domain 2 (SD2) in the spike protein of the COVID-19 virus may include the nucleotide sequence of SEQ ID NO: 3, and specifically, the gene may include a nucleotide sequence having a sequence homology of 70% or more, more preferably, 80% or more, still more preferably, 90% or more, and most preferably, 95% or more to the nucleotide sequence of SEQ ID NO: 3. The "% of sequence homology" to a polynucleotide is determined by comparing two optimally aligned sequences with a comparison region, and a portion of the polynucleotide sequence in the comparison region may include additions or deletions (*i.e*., gaps) compared to a reference sequence (not including additions or deletions) to the optimal alignment of the two sequences.

In the spike protein of the COVID-19 virus, the S2 protein may include the amino acid sequence of SEQ ID NO: x.

The protein of the trimeric motif region of Coronin 1 (mCor 1) may include the amino acid sequence of SEQ ID NO: 6.

The gene encoding the protein of the trimeric motif region of Coronin 1 (mCor1) may include the nucleotide sequence of SEQ ID NO: 5, and specifically, the gene may include a nucleotide sequence having a sequence homology of 70% or more, more preferably, 80% or more, still more preferably, 90% or more, and most preferably, 95% or more to the nucleotide sequence of SEQ ID NO: 5. The "% of sequence homology" to a polynucleotide is determined by comparing two optimally aligned sequences with a comparison region, and a portion of the polynucleotide sequence in the comparison region may include additions or deletions (*i.e*., gaps) compared to a reference sequence (not including additions or deletions) to the optimal alignment of the two sequences.

The recombinant genes were introduced into a plant expression vector, pTEX1, to make a plant expression vector. Six types of ectodomain recombinant protein genes were constructed from the spike gene of COVID-19 (FIG. 1), and the expression of the recombinant protein was induced by introducing them into plants (*Nicotiana benthamiana*)*.* In order to confirm the expression of the protein in the leaf extract of *N. benthamiana* into which these genes were introduced, Western blot analysis was performed by using an anti-His antibody. As shown in FIG. 2, S_{full} was confirmed at about 180 kD. Since it forms N-glycosylation of the spike protein, it was determined that it appeared to be larger than the calculated protein position. S 1 and S2 appeared at the positions of 100 kD and 75 kD, respectively, and since these two proteins also appeared to be larger than the calculated size, it was also determined to be N-glycosylation (FIG. 2).

Further, in order to isolate these two types of proteins purely, a total extract of *N. benthamiana* was prepared, and pure isolation and purification were performed by using Ni²⁺-NTA affinity column chromatography therefrom, and afterwards, it was isolated and purified by SDS/PAGE and stained by Coomassie brilliant blue. Through this, it was confirmed that the recombinant proteins of the two types of the spike proteins could be purely isolated and purified (FIG. 3).

The recombinant vector may further include any one promoter selected from the group consisting of a 35S promoter derived from cauliflower mosaic virus, a 19S RNA promoter derived from cauliflower mosaic virus, a Mac promoter, an actin protein promoter and ubiquitin protein promoter of a plant, preferably, it may be a Mac promoter, and more preferably, a MacT promoter.

The MacT promoter may be a promoter in which A, which is the 3' terminal nucleotide of the Mac promoter nucleotide sequence, is substituted with T, and the MacT promoter may include the nucleotide sequence of SEQ ID NO: 17, and specifically, the gene may include a nucleotide sequence having a sequence homology of 70% or more, more preferably, 80% or more, still more preferably, 90% or more, and most preferably, 95% or more to the nucleotide sequence of SEQ ID NO: 17. The "% of sequence homology" to a polynucleotide is determined by comparing two optimally aligned sequences with a comparison region, and a portion of the polynucleotide sequence in the comparison region may include additions or deletions (*i.e.,* gaps) compared to a reference sequence (not including additions or deletions) to the optimal alignment of the two sequences.

The recombinant vector may further include an RD29B-t termination site, and the RD29B-t termination site may include the nucleotide sequence of SEQ ID NO: 18, and specifically, the gene may include a nucleotide sequence having a sequence homology of 70% or more, more preferably, 80% or more, still more preferably, 90% or more, and most preferably, 95% or more to the nucleotide sequence of SEQ ID NO: 18. The "% of sequence homology" to a polynucleotide is determined by comparing two optimally aligned sequences with a comparison region, and a portion of the polynucleotide sequence in the comparison region may include additions or deletions (*i.e*., gaps) compared to a reference sequence (not including additions or deletions) to the optimal alignment of the two sequences.

By including the BiP signal sequence and the ER retention signal HDEL at the N-terminus and C-terminus of the recombinant protein gene, respectively, it may have an effect of inducing accumulation in the endoplasmic reticulum (ER) at a high concentration. The recombinant vector may further include any one selected from the group consisting of a chaperone binding protein (BiP) and a His-Asp-Glu-Leu (HDEL) peptide, and the chaperone binding protein (BiP) may include the nucleotide sequence of SEQ ID NO: 12, and HDEL (His-Asp-Glu-Leu) may include the nucleotide sequence of SEQ ID NO: 10.

The recombination refers to a cell in which the cell replicates a heterologous nucleic acid, expresses the nucleic acid, or expresses a peptide, a heterologous peptide or a protein encoded by the heterologous nucleic acid. Recombinant cells can express genes or gene segments that are not found in the native form of the cell in either the sense or antisense form. In addition, recombinant cells can express genes found in the native form of cells, but the genes are modified and re-introduced into cells by artificial means.

The term "recombinant expression vector" means a bacterial plasmid, phage, yeast plasmid, plant cell virus, mammalian cell virus or other vectors. In general, any plasmid and vector may be used as long as it is capable of replication and stabilization in the host. An important characteristic of the expression vector is that it has an origin of replication, a promoter, a marker gene and a translation control element. The recombinant expression vector and the expression vector including appropriate transcriptional/translational control signals may be constructed by a method well known to those skilled in the art. The method includes *in vitro* recombinant DNA technology, DNA synthesis technology and *in vivo* recombination technology.

A preferred example of the recombinant vector of the present invention is a Ti-plasmid vector capable of transferring a part of itself, the so-called T-region, into a plant cell when present in a suitable host. Another type of the Ti-plasmid vector is currently being used to transfer hybrid DNA sequences into plant cells, or protoplasts from which new plants can be produced that properly insert the hybrid DNA into the genome of the plant. A particularly preferred form of the Ti-plasmid vector is the so-called binary vector as claimed in EP 0120 516 B1 and US Patent. No. 4,940,838. Other suitable vectors that can be used to introduce the DNA according to the invention into a plant host may be selected from viral vectors such as those that can be derived from double-stranded plant viruses (*e.g.*, CaMV) and single-stranded viruses, geminiviruses and the like, and for example, incomplete plant viral vectors. The use of such vectors may be advantageous, especially when it is difficult to adequately transform a plant host.

In addition, the present invention may provide a transgenic organism which is transformed by the recombinant vector.

The transformed transgenic organism may be a prokaryote or a eukaryote, and for example, yeast (*Saccharomyce cerevisiae*)*,* fungi such as *E. coli,* insect cells, human cells (e.g., CHO cell line (Chinese hamster ovary), W138, BHK, COS-7, 293, HepG2, 3T3, RIN and MDCK cell lines) and plant cells may be used, and preferably it may be *Agrobacterium.* In the case of insect cells and human cells, the gene encoding a recombinant spike protein forming a trimer may be expressed by using an expression vector necessary for the expression of each of these types of animal cells.

Methods for delivering the vector of the present invention into a host cell may be carried out by the CaCl₂ method, the Hanhan method (Hanahan, D., J. Mol. Biol., 166:557-580 (1983)), the electroporation method or the like, when the host cell is a prokaryotic cell. In addition, when the host cell is a eukaryotic cell, the vector may be injected into the host cell by microinjection, calcium phosphate precipitation, electroporation, liposome-mediated transfection, DEAE-dextran treatment, gene bombardment and the like.

The present invention may provide a method for producing a recombinant spike protein of a coronavirus forming a trimer in a plant, including the steps of:
(a) constructing the aforementioned recombinant vector;
(b) preparing a transgenic organism by introducing the recombinant vector into an organism;
(c) culturing the transgenic organism;
(d) infiltrating the culture product into a plant; and
(e) pulverizing the plant to obtain a recombinant spike protein of a coronavirus forming a trimer.

The method of infiltrating the plant may include a chemical cell method, a vacuum or syringe infiltration method, and most preferably, it may be a syringe infiltration method, but is not limited thereto.

The plant may be selected from food crops including rice, wheat, barley, corn, soybean, potato, wheat, red bean, oat and sorghum; vegetable crops including *Arabidopsis thaliana,* Chinese cabbage, radish, red pepper, strawberry, tomato, watermelon, cucumber, cabbage, Korean melon, pumpkin, green onion, onion and carrot; special crops including ginseng, tobacco, cotton, sesame, sugar cane, sugar beet, perilla, peanut and rapeseed; fruit trees including apple tree, pear tree, date tree, peach, grape, tangerine, persimmon, plum, apricot and banana; and flowers including rose, carnation, chrysanthemum, lily and tulip.

The spike proteins of the COVID-19 virus produced in this way may be isolated and purified by Ni²⁺-NTA affinity column chromatography using the C-terminal His tag. In addition, the isolated protein may be further isolated by using size exclusion gel chromatography to increase the purity.

Another object of the present invention is to provide a vaccine composition using the spike protein of these plant-produced COVID-19. S_{full-t} or S2s-t of the trimer of the spike protein may be used with or without alum to constitute a vaccine composition.

### [Modes of the Invention]

Hereinafter, preferred examples are presented to help the understanding of the present invention. However, the following examples are only provided for easier understanding of the present invention, and the contents of the present invention are not limited by the following examples.

### <Example 1> Design of gene encoding recombinant spike protein of COVID-19 virus forming trimer

In order to induce the expression of the spike (S) derived from the COVID-19 virus surface protein in a soluble form in the ER lumen, the ectodomain of the spike protein with the transmembrane domain to the C-terminus removed (from the 16^{th} amino acid to the 1,213^{th} amino acid, a total of 1,198 residues, SfΔ(TMD-CT)), or an S1 subunit region including the N-terminus to the SD2 domain (from the 16^{th} amino acid to the 681^{st} amino acid, a total of 666 residues), or the ectodomain of S2 (the 682^{nd} amino acid to the 1,213^{th} amino acid, a total of 532 residues) was obtained. The ER targeting signal obtained from BiP, which is a protein from *Arabidopsis thaliana,* was fused to the 5'-end of the spark gene of the COVID-19 virus to enable ER targeting. Further, in order to induce trimer formation of the recombinant spike protein thus made, a Hisx5 tag and HDEL, which is an ER retention motif, were sequentially fused to the C-terminus of mCor1 for the purification of the spike protein and high accumulation in the ER to construct a construct. For expression, MacT was used, and the end of Rd29b was used as a transcription terminator. The transcription terminator was confirmed to show high transcription efficiency as a result of previous studies. The nucleotide sequences used in the experiment are shown in Table 1 below (FIG. 1).

Then, these constructs were introduced into pTEX to construct 9 final plant expression vectors.

**[Table 1]**

| | Sequence (5'-3) | |
|---|---|---|
| Nucleotide sequence of SfΔ(TMD-CT) | gttaaccttaccaccagaactcagttacccccagcatacactaattctttcacacgtggtgtttactaccctgacaaagtt ttcagaagcagcgttttacacagcactcaggatttattcctacctttcttttccaacgtgacctggttccatgctatacatg tatctgggaccaatggtaccaagaggtttgataacccggtcctaccatttaatgatggagtctattttgcctccactgag aagtctaatataataagaggctggatttttggaactactcttgattcgaagacccagagtctacttattgttaataacgct acaaatgttgttatcaaagtatgtgaatttcaattctgtaatgatccattcttgggtgtttactaccacaaaaacaacaaaa gttggatggaaagtgagtttcgggtttatagcagtgcgaataattgcacttttgagtacgtctcccaaccttttcttatgg accttgaaggaaagcagggaaatttcaagaatcttcgcgaatttgtgtttaagaatatcgatggttatttcaagatatatt ctaagcacacgcctattaatttagtgcgagatctccctcagggtttttcggcgctggaaccattggtagatttgccgata ggaatcaatatcactaggttccagactttacttgctctgcatagaagttacttgacccctggagatagctcatcaggttg gacagctggtgcggcagcttattacgtggggtatcttcagcctaggacgttcctattaaaatataatgaaaatggaac cattacagatgctgtagactgtgcacttgaccctctctcagaaacaaagtgtacgttgaaatccttcacggtagaaaaa gggatctaccaaacgtctaacttcagagtccagccaacagaatctattgtgagatttcccaatattacaaacttgtgcc ctttcggagaagtttttaacgccaccaggtttgcatcggtttatgcttggaacaggaaaagaatcagcaactgtgttgct gattatagtgtcctatataactccgcatccttttccactttcaagtgttacggagtttctcctactaaattaaatgatctctgc tttactaatgtctatgcagattcatttgtaatcagaggtgatgaggtcagacaaatcgctccagggcagactggaaag attgctgattataattataagcttcctgatgattttacaggctgcgttatagcatggaattctaataatcttgactctaaggt ggggggaaattataattacctgtatagactgtttaggaagagcaatctcaagcctttcgagagagacatttcaactga gatctaccaggcgggaagcactccgtgtaatggtgttgagggttttaattgttactttcctttacagtcatacggtttcca | SEQ ID NO: 1 |
| | acccacgaatggggttggttaccaaccgtaccgagtagtagtactttctttcgagcttctacatgccccagcaactgttt gtggacctaagaagtctactaatttggttaaaaataagtgtgtcaattttaatttcaatggacttacgggcacaggagttc ttactgagtctaacaagaagtttctgcctttccagcagttcggcagagatattgctgacactactgatgctgtgcgtgat ccacagacacttgaaattcttgacattacaccatgttcttttggtggcgtgagtgttataactcccggaacaaatacctc caaccaggtggctgttctgtatcaggacgtgaactgtacagaagtccctgttgcaattcatgcagatcagcttactcct acctggcgtgtttattctacgggttccaatgtttttcaaacacgtgcaggctgcttgataggggctgaacatgtcaacaa ctcatatgaatgcgacatacccataggtgcaggtatatgcgctagttatcagactcagaccaattctccgcggcgggc acgaagtgtagctagtcaatccatcattgcctacactatgtcacttggtgcagaaaattcagttgcttactctaataactc tattgccatacccacaaattttactattagtgttaccacagaaattctaccagtgtctatgaccaagacatcagttgactgt acaatgtatatttgcggggattcaactgagtgctcgaatctgttgttgcaatacggcagtttttgtacccaattgaaccg ggctctgactggaatagctgtggaacaagataaaaacacccaagaagtttttgcacaagtcaaacaaatttataaaac accaccaattaaagatttcggtggtttcaacttctcacaaatactgccagatccgagcaaaccaagcaagaggtcatt cattgaagacctacttttcaacaaagtgacacttgcagatgctggcttcattaaacagtatggtgattgcttgggggata ttgctgctagagacctcatttgtgcacaaaagtttaacgggctgacagtgttgccacctttgttgacagatgagatgatt gctcagtacacttctgcactgctcgctggtacaatcacatctgggtggacctttggtgcaggtgctgccttacaaatac catttgctatgcagatggcttataggttcaatggtatcggagttacacagaacgttctctatgagaaccaaaaattgatt gccaaccaattcaatagtgccattggcaagattcaggactcactttcaagcacagcgagtgcacttggaaagttgca agatgtggtcaaccagaatgcacaagctttaaacacgcttgtgaaacaactcagctccaactttggggcaatttcaag tgttttgaatgatatcctttcacgtcttgataaagtggaagccgaggtgcaaattgacaggttgatcacaggccgacttc aaagtttgcagacttatgtgactcaacaattaattagggcagcagaaatccgcgcttcggctaatctggcggctacta aaatgtcagagtgtgtacttggacaatctaaacgagttgatttttgcggaaagggctatcatctcatgtccttccctcagt cagcgcctcacggtgtagtgttcttgcacgtgacttacgttcctgcacaagaaaagaatttcacaactgctccggccat ttgtcatgatggaaaagcccactttccgcgtgaaggtgtctttgtttcgaatggcacacactggtttgtaacccaaagg aatttttatgagccacaaatcattacgacggacaacacttttgtgtctggtaattgtgatgttgtaatcggaatcgtcaac aacaccgtttacgatcctttgcagcctgagttagattctttcaaagaggagctggataagtatttcaagaatcatacatc acccgatgttgatctcggtgatatctctggaattaatgcttcagttgtgaacattcaaaaggagattgaccgcctcaatg aggttgccaagaatttgaatgaatcgctcatcgatctccaagaacttggaaagtatgagcagtatatcaagtggcca | |
| Amino acid sequence of SfΔ(TMD-CT) | vnlttrtqlppaytnsftrgvyypdkvfrssvlhstqdlflpffsnvtwfhaihvsgtngtkrfdnpvlpfndgvyfa steksniirgwifgttldsktqsllivnnatnvvikvcefqfcndpflgvyyhknnkswmesefrvyssannctfe yvsqpflmdlegkqgnfknlrefvfknidgyfkiyskhtpinlvrdlpqgfsaleplvdlpiginitrfqtllalhrs yltpgdsssgwtagaaayyvgylqprtfllkynengtitdavdcaldplsetkctlksftvekgiyqtsnfrvqpte sivrfpnitnlcpfgevfnatrfasvyawnrkrisncvadysvlynsasfstfkcygvsptklndlcftnvyadsfv irgdevrqiapgqtgkiadynyklpddftgcviawnsnnldskvggnynylyrlfrksnlkpferdisteiyqag stpcngvegfncyfplqsygfqptngvgyqpyrvvvlsfellhapatvcgpkkstnlvknkcvnfnfngltgtg vltesnkkflpfqqfgrdiadttdavrdpqtleilditpcsfggvsvitpgtntsnqvavlyqdvnctevpvaihad qltptwrvystgsnvfqtragcligaehvnnsyecdipigagicasyqtqtnsprrarsvasqsiiaytmslgaens vaysnnsiaiptnftisvtteilpvsmtktsvdctmyicgdstecsnlllqygsfctqlnraltgiaveqdkntqevfa | SEQ ID NO: 2 |
| | qvkqiyktppikdfggfnfsqilpdpskpskrsfiedllfnkvtladagfikqygdclgdiaardlicaqkfngltvl pplltdemiaqytsallagtitsgwtfgagaalqipfamqmayrfngigvtqnvlyenqklianqfnsaigkiqds lsstasalgklqdvvnqnaqalntlvkqlssnfgaissvlndilsrldkveaevqidrlitgrlqslqtyvtqqliraae irasanlaatkmsecvlgqskrvdfcgkgyhlmsfpqsaphgvvflhvtyvpaqeknfttapaichdgkahfpr egvfvsngthwfvtqrnfyepqiittdntfvsgncdvvigivnntvydplqpeldsfkeeldkyfknhtspdvdl gdisginasvvniqkeidrlnevaknlneslidlqelgkyeqyikwp | |
| Nucleotide sequence of S1s | gttaaccttaccaccagaactcagttacccccagcatacactaattctttcacacgtggtgtttactaccctgacaaagtt ttcagaagcagcgttttacacagcactcaggatttattcctacctttcttttccaacgtgacctggttccatgctatacatg tatctgggaccaatggtaccaagaggtttgataacccggtcctaccatttaatgatggagtctattttgcctccactgag aagtctaatataataagaggctggatttttggaactactcttgattcgaagacccagagtctacttattgttaataacgct acaaatgttgttatcaaagtatgtgaatttcaattctgtaatgatccattcttgggtgtttactaccacaaaaacaacaaaa gttggatggaaagtgagtttcgggtttatagcagtgcgaataattgcacttttgagtacgtctcccaaccttttcttatgg accttgaaggaaagcagggaaatttcaagaatcttcgcgaatttgtgtttaagaatatcgatggttatttcaagatatatt ctaagcacacgcctattaatttagtgcgagatctccctcagggtttttcggcgctggaaccattggtagatttgccgata ggaatcaatatcactaggttccagactttacttgctctgcatagaagttacttgacccctggagatagctcatcaggttg gacagctggtgcggcagcttattacgtggggtatcttcagcctaggacgttcctattaaaatataatgaaaatggaac cattacagatgctgtagactgtgcacttgaccctctctcagaaacaaagtgtacgttgaaatccttcacggtagaaaaa gggatctaccaaacgtctaacttcagagtccagccaacagaatctattgtgagatttcccaatattacaaacttgtgcc ctttcggagaagtttttaacgccaccaggtttgcatcggtttatgcttggaacaggaaaagaatcagcaactgtgttgct gattatagtgtcctatataactccgcatccttttccactttcaagtgttacggagtttctcctactaaattaaatgatctctgc tttactaatgtctatgcagattcatttgtaatcagaggtgatgaggtcagacaaatcgctccagggcagactggaaag attgctgattataattataagcttcctgatgattttacaggctgcgttatagcatggaattctaataatcttgactctaaggt ggggggaaattataattacctgtatagactgtttaggaagagcaatctcaagcctttcgagagagacatttcaactga gatctaccaggcgggaagcactccgtgtaatggtgttgagggttttaattgttactttcctttacagtcatacggtttcca acccacgaatggggttggttaccaaccgtaccgagtagtagtactttctttcgagcttctacatgccccagcaactgttt gtggacctaagaagtctactaatttggttaaaaataagtgtgtcaattttaatttcaatggacttacgggcacaggagttc ttactgagtctaacaagaagtttctgcctttccagcagttcggcagagatattgctgacactactgatgctgtgcgtgat ccacagacacttgaaattcttgacattacaccatgttcttttggtggcgtgagtgttataactcccggaacaaatacctc caaccaggtggctgttctgtatcaggacgtgaactgtacagaagtccctgttgcaattcatgcagatcagcttactcct acctggcgtgtttattctacgggttccaatgtttttcaaacacgtgcaggctgcttgataggggctgaacatgtcaacaa ctcatatgaatgcgacatacccataggtgcaggtatatgcgctagttatcagactcagaccaattctccg | SEQ ID NO: 3 |
| Amino acid sequence of S1s protein | vnlttrtqlppaytnsftrgvyypdkvfrssvlhstqdlflpffsnvtwfhaihvsgtngtkrfdnpvlpfndgvyfa steksniirgwifgttldsktqsllivnnatnvvikvcefqfcndpflgvyyhknnkswmesefrvyssannctfe yvsqpflmdlegkqgnfknlrefvfknidgyfkiyskhtpinlvrdlpqgfsaleplvdlpiginitrfqtllalhrs yltpgdsssgwtagaaayyvgylqprtfllkynengtitdavdcaldplsetkctlksftvekgiyqtsnfrvqpte sivrfpnitnlcpfgevfnatrfasvyawnrkrisncvadysvlynsasfstfkcygvsptklndlcftnvyadsfv irgdevrqiapgqtgkiadynyklpddftgcviawnsnnldskvggnynylyrlfrksnlkpferdisteiyqag | SEQ ID NO: 4 |
| | stpcngvegfncyfplqsygfqptngvgyqpyrvvvlsfellhapatvcgpkkstnlvknkcvnfnfngltgtg vltesnkkflpfqqfgrdiadttdavrdpqtleilditpcsfggvsvitpgtntsnqvavlyqdvnctevpvaihad qltptwrvystgsnvfqtragcligaehvnnsyecdipigagicasyqtqtnsp | |
| Nucleotide sequence of S2sΔ (TMD-CT) | cggcgggcacgaagtgtagctagtcaatccatcattgcctacactatgtcacttggtgcagaaaattcagttgcttact ctaataactctattgccatacccacaaattttactattagtgttaccacagaaattctaccagtgtctatgaccaagacatc agttgactgtacaatgtatatttgcggggattcaactgagtgctcgaatctgttgttgcaatacggcagtttttgtaccca attgaaccgggctctgactggaatagctgtggaacaagataaaaacacccaagaagtttttgcacaagtcaaacaaa tttataaaacaccaccaattaaagatttcggtggtttcaacttctcacaaatactgccagatccgagcaaaccaagcaa gaggtcattcattgaagacctacttttcaacaaagtgacacttgcagatgctggcttcattaaacagtatggtgattgctt gggggatattgctgctagagacctcatttgtgcacaaaagtttaacgggctgacagtgttgccacctttgttgacagat gagatgattgctcagtacacttctgcactgctcgctggtacaatcacatctgggtggacctttggtgcaggtgctgcct tacaaataccatttgctatgcagatggcttataggttcaatggtatcggagttacacagaacgttctctatgagaaccaa aaattgattgccaaccaattcaatagtgccattggcaagattcaggactcactttcaagcacagcgagtgcacttgga aagttgcaagatgtggtcaaccagaatgcacaagctttaaacacgcttgtgaaacaactcagctccaactttggggc aatttcaagtgttttgaatgatatcctttcacgtcttgataaagtggaagccgaggtgcaaattgacaggttgatcacag gccgacttcaaagtttgcagacttatgtgactcaacaattaattagggcagcagaaatccgcgcttcggctaatctgg cggctactaaaatgtcagagtgtgtacttggacaatctaaacgagttgatttttgcggaaagggctatcatctcatgtcc ttccctcagtcagcgcctcacggtgtagtgttcttgcacgtgacttacgttcctgcacaagaaaagaatttcacaactg ctccggccatttgtcatgatggaaaagcccactttccgcgtgaaggtgtctttgtttcgaatggcacacactggtttgta acccaaaggaatttttatgagccacaaatcattacgacggacaacacttttgtgtctggtaattgtgatgttgtaatcgg aatcgtcaacaacaccgtttacgatcctttgcagcctgagttagattctttcaaagaggagctggataagtatttcaaga atcatacatcacccgatgttgatctcggtgatatctctggaattaatgcttcagttgtgaacattcaaaaggagattgacc gcctcaatgaggttgccaagaatttgaatgaatcgctcatcgatctccaagaacttggaaagtatgagcagtatatcaa gtggcca | SEQ ID NO: 5 |
| Amino acid sequence of S2sΔ (TMD-CT) protein | RRARSVASQSIIAYTMSLGAENSVAYSNNSIAIPTNFTISVTTEILPVSMTKT SVDCTMYICGDSTECSNLLLQYGSFCTQLNRALTGIAVEQDKNTQEVFAQ VKQIYKTPPIKDFGGFNFSQILPDPSKPSKRSFIEDLLFNKVTLADAGFIKQ YGDCLGDIAARDLICAQKFNGLTVLPPLLTDEMIAQYTSALLAGTITSGW TFGAGAALQIPFAMQMAYRFNGIGVTQNVLYENQKLIANQFNSAIGKIQD SLSSTASALGKLQDVVNQNAQALNTLVKQLSSNFGAISSVLNDILSRLDK VEAEVQIDRLITGRLQSLQTYVTQQLIRAAEIRASANLAATKMSECVLGQ SKRVDFCGKGYHLMSFPQSAPHGVVFLHVTYVPAQEKNFTTAPAICHDG KAHFPREGVFVSNGTHWFVTQRNFYEPQIITTDNTFVSGNCDVVIGIVNN TVYDPLQPELDSFKEELDKYFKNHTSPDVDLGDISGINASVVNIQKEIDRL NEVAKNLNESLIDLQELGKYEQYIKWP | SEQ ID NO: 6 |
| Nucleotide sequence of | gtgtctaggcttgaggaagatgttagaaatctcaacgcaattgtccagaaacttcaggaaaggttggataggctgga ggaaactgttcaagctaag | SEQ ID NO: 7 |
| mCOr1 | | |
| Amino acid sequence of mCor1 | vsrleedvrnlnaivqklqerldrleetvqak | SEQ ID NO: 8 |
| Nucleotide sequence of Linker | ggaggttcaggaggttcaggaggttcaggaggttca | SEQ ID NO: 9 |
| Amino acid sequence of Linker | ggsggsggsggs | SEQ ID NO: 10 |
| Nucleotide sequence of BiP | atggctcgct cgtttggagc taacagtacc gttgtgttgg cgatcatctt cttcggtgag tgattttccg atcttcttct ccgatttaga tctcctctac attgttgctt aatctcagaa ccttttttcg ttgttcctgg atctgaatgt gtttgtttgc aatttcacga tcttaaaagg ttagatctcg attggtattg acgattggaa tctttacgat ttcaggatgt ttatttgcgt tgtcctctgc aatagaagag gctacgaagt ta | SEQ ID NO: 11 |
| Nucleotide sequence of HDEL | cacgatgagctc | SEQ ID NO: 12 |
| Amino acid sequence of HDEL | His-Asp-Glu-Leu | SEQ ID NO: 13 |
| Nucleotide sequence of 5'UTR | aattattacatcaaaacaaaaa | SEQ ID NO: 14 |
| Nucleotide sequence of MacT | agagatctcctttgccccagagatcacaatggacgacttcctctatctctacgatctagtcaggaagttcgacggaga aggtgacgataccatgttcaccactgataatgagaagattagccttttcaatttcagaaagaatgctaacccacagatg gttagagaggcttacgcagcaggtctcatcaagacgatctacccgagcaataatctccaggagatcaaataccttcc caagaaggttaaagatgcagtcaaaagattcaggactaactgcatcaagaacacagagaaagatatatttctcaaga tcagaagtactattccagtatggacgattcaaggcttgcttcacaaaccaaggcaagtaatagagattggagtctctaa aaaggtagttcccactgaatcaaaggccatggagtcaaagattcaaatagaggacctaacagaactcgccgtaaag actggcgaacagttcatacagagtctcttacgactcaatgacaagaagaaaatcttcgtcaacatggtggagcacga cacgcttgtctactccaaaaatatcaaagatacagtctcagaagaccaaagggcaattgagacttttcaacaaagggt aatatccggaaacctcctcggattccattgcccagctatctgtcactttattgtgaagatagtggaaaaggaaggtggc tcctacaaatgccatcattgcgataaaggaaaggccatcgttgaagatgcctctgccgacagtggtcccaaagatgg acccccacccacgaggagcatcgtggaaaaagaagacgttccaaccacgtcttcaaagcaagtggattgatgtga cgcaagacgtgacgtaagtatctgagctagtttttatttttctactaatttggtcgtttatttcggcgtgtaggacatggca accgggcctgaatttcgcgggtattctgtttctattccaactttttcttgatccgcagccattaacgacttttgaatagata cgctgacacgccaagcctcgctagtcaaaagtgtaccaaacaacgctttacagcaagaacggaatgcgcgtgacg | SEQ ID NO: 15 |
| | ctcgcggtgacgccatttcgccttttcagaaatggataaatagccttgcttcctattatatcttcccaaattaccaatacat tacactagcatctgaatttcataaccaatctcgatacaccaaatcgt | |
| Nucleotide sequence of RD29B terminator | aattttactcaaaatgttttggttgctatggtagggactatggggttttcggattccggtggaagtgagtggggaggca gtggcggaggtaagggagttcaagattctggaactgaagatttggggttttgcttttgaatgtttgcgtttttgtatgatg cctctgtttgtgaactttgatgtattttatctttgtgtgaaaaagagattgggttaataaaatatttgcttttttggataagaaa ctcttttagcggcccattaataaaggttacaaatgcaaaatcatgttagcgtcagatatttaattattcgaagatgattgtg [?]atagatttaaaattatcctagtcaaaaagaaagagtaggttgagcagaaacagtgacatctgttgtttgtaccatacaaa ttagtttagattattggttaacatgttaaatggctatgcatgtgacatttagaccttatcggaattaatttgtagaattattaat taagatgttgattagttcaaacaaaaat | SEQ ID NO: 16 |

### <Example 2> Expression and confirmation of gene encoding recombinant spike protein of COVID-19 virus forming trimer

6 types of constructs shown in FIG. 1 [SfΔ(TMD-CT):mCor1:Hisx5:HDEL (S_{full}-t), SfΔ(TMD-CT):Hisx5:HDEL (S_{full-m}), S1s:mCor1:Hisx5:HDEL (S1s-t), S1s:Hisx5:HDEL (S1s-m), S2sΔ(TMD-CT):mCor1:Hisx5:HDEL (S2s-t), S2sΔ(TMD-CT):Hisx5:HEDL (S2s-m)] were induced for expression through transient expression in 4 to 5 week-old *Nicotiana benthamiana* plant leaves by using the vacuum infiltration method. Infiltrated leaves were harvested 5 days after infiltration (dpi), thoroughly ground in liquid nitrogen and dissolved in 3 volumes of buffer. The total soluble protein from the infiltrated leaf extract was developed by SDS-PAGE, followed by Western blot analysis. In the spike (S) recombinant proteins, S_{full -t} and S_{full-m} were identified at about the 180 kD position, and S1s-t and S1s-m were identified at the 100 kD position by an anti-His antibody. In addition, S2s-t and S2s-m were identified at the 75 kD position. Comparing the positions of the constructs without mCor and the proteins with mCor1, it can be seen that the difference between them was due to mCor1, as those with mCor1 were slightly smaller than those without mCor1. In addition, since all of these six recombinant proteins appeared to be larger than the calculated sizes, it was determined that this was due to N-glycosylation (FIG. 2). In addition, when the bands were confirmed by staining the same membrane with Coomassie Brilliant Blue, they were also observed here. Judging from the band intensity, the expression level of these recombinant proteins was estimated to be on the order of 20 to 50 µg/g fresh weight in infiltrated leaves.

### <Example 3> Isolation and purification of recombinant spark protein of COVID-19 virus forming trimer

S_{full-t} and S2s-t, which are the recombinant proteins of two types of COVID-19 virus S protein, were expressed in *N. benthamiana,* isolated and purified by Ni²⁺-NTAaffinity column, and developed through SDS/PAGE, and afterwards, Western blot was performed using an anti-His antibody to confirm isolation and purification. Subsequently, the membrane was stained with Coomassie brilliant blue. Through this, it was confirmed that the recombinant proteins derived from the two types of spark proteins could be purified by pure isolation (FIG. 3).

### <Example 4> Isolation and purification of S_{full-t} recombinant protein forming trimer through size exclusion column chromatography

The recombinant protein of S_{full-t} isolated and purified by Ni²⁺-NTA affinity column was concentrated, and it was isolated and purified again by using size exclusion column chromatography. The fractions obtained from the column were developed through SDS/PAGE from No. 7 to No. 20, and Western blot was performed using an anti-His antibody to confirm the location and elution amount of the Se recombinant protein. Afterwards, the membrane was stained with Coomassie brilliant blue to confirm the contamination of other protein bands (FIG. 4).

### <Example 5> Confirmation of difference in fractionation through size exclusion column of recombinant protein with and without mCor1 of SfΔ (TMD-CT) of COVID-19 virus and trimer formation of S_{full-t} recombinant protein by mCor1

After isolating and purifying proteins by Ni²⁺-NTA affinity column from *N*. *benthamiana* leaf extracts expressing S_{full-t} and S_{full-m}, respectively (FIG. 5A and B), these proteins were analyzed by using size exclusion column chromatography, and after fractionation (FIG. 6C), these fractions were purified using SDS/PAGE and analyzed by Western blotting using an anti-His antibody (FIG. 5D). After negative staining of the isolated S_{full-t} protein, the shape of the protein was observed using an electron microscope to confirm the formation of a trimer (FIG. 5E).

### <Example 6> Isolation and purification of recombinant protein of S2e forming trimer through size exclusion column chromatography

The recombinant protein of S2s-t isolated and purified by Ni²⁺-NTA affinity column was concentrated, and it was isolated and purified again by using size exclusion column chromatography. The fractions obtained from the column were developed through SDS/PAGE from No. 7 to No. 20, and Western blot was performed using an anti-His antibody to confirm the location and elution amount of the S2s-t recombinant protein (FIG. 6).

### <Example 7> Immunogenicity evaluation in experimental animals of COVID-19 plant vaccine candidates

### (1) Evaluation of immunogenicity in experimental animals (mouse, Balb/c) of COVID-19 plant vaccine candidates

For animal experiments to evaluate the immunogenicity of the prepared plant vaccine, 6-week-old female mice (Balb/c) were used, 5 mice were assigned per experimental group, and four doses (1, 5, 15, 30 µg) were carried out for immunization. Each immune antigen (S_{full-t}) was immunized 3 times at intervals of 2 weeks by an intramuscular route, and in order to confirm the production of antibodies to the immune antigen, blood was collected before immunization, 2 weeks after primary immunization, secondary immunization and tertiary immunization to carry out the evaluation of immunogenicity such as antibody (IgGs) production and neutralizing antibody production in serum. In this case, the negative control group was immunized with PBS.

### 1) Humoral immune response analysis

In the evaluation of the immunogenicity of the prepared plant vaccine, it was confirmed through ELISA whether antigen-specific antibodies were generated in the serum. It was confirmed that sufficient IgG titers appeared after a total of 2 or 3 mouse immunizations, and it was confirmed that both S 1 and S2 specific antibodies, which are subdomains of the S antigen, were generated by this vaccine, and it was particularly confirmed that the titer against S2 was high. In addition, it was confirmed that the group immunized with an immune adjuvant (aluminum hydroxide) at the same time showed somewhat higher IgG titers and neutralizing antibody titers compared to the group not administered simultaneously.

### 2) Neutralizing antibody induction analysis

After separating the serum from the blood of the mice immunized with the prepared plant vaccine, the plaque reduction neutralization test (PRNT), which is generally known as the "gold standard" among the methods of measuring virus-neutralizing antibodies, was used to confirm the neutralizing ability of the antibody against SARS-CoV-2. In order to analyze the neutralizing antibody titer, the virus was treated in mouse serum (antibody) by using SARS-CoV-2 virus (BetaCoV/Korea/KCDC03/2020) received from the National Culture Collection for Pathogens, and afterwards, the change in the infection rate was confirmed. In addition, cross-reactions analysis for the recently reported UK mutant (B.1.1.7) and South African mutant (B. 1.351) was also performed.

The neutralizing antibody induction showed higher neutralizing antibody titers in the group immunized with the immune adjuvant at the same time than the group injected with the immune antigen alone, similar to the result of IgG production, and it was confirmed that a higher neutralizing antibody titer was shown in the serum collected after immunization at a high concentration than in the serum immunized with a low concentration. In addition, it was confirmed that neutralizing antibodies were not induced in the PBS group, which was a negative control group (FIG. 7C). In addition, it was confirmed that cross-reactions also appeared in the UK and South African mutants, which have recently become a problem (FIG. 7D).

### 3) Cell-mediated immune response analysis

Cell-mediated immune response according to plant vaccine immunization was analyzed using the ELISPOT test method. ELISPOST analysis was performed using a commercial IFN-γ ELISPOT kit. In the cell-mediated immune response, the number of splenocytes secreting IFN-γ was increased in the group immunized with the plant vaccine compared to the control group (PBS group), and it was confirmed that it was not significantly proportional to the amount of antigen. In addition, there was no increase effect according to the immune adjuvant. In conclusion, it could be confirmed that the cellular immune response was induced together with the humoral immunity by plant vaccine immunization (FIG. 7E).

### <Example 8> Evaluation of protective efficacy in experimental animals (TG mice) of COVID-19 plant vaccine candidates

An animal experiment to evaluate the protective efficacy of the plant vaccine prepared according to the present invention was performed by using 6-week-old transgenic mice (B6.Cg-Tg(K18-ACE2)2Primn/J) expressing the SARS-CoV-2 human receptor ACE2, and 14 mice were assigned per experimental group (12 mice in the control group), and immunization was performed at two doses (15 and 30 µg). Each immune antigen was immunized twice at intervals of 2 weeks by intramuscular route, and in order to confirm the production of antibodies to the immune antigen, blood was collected before immunization, 2 weeks after primary immunization and 2 weeks after secondary immunization to perform the evaluation of immunogenicity such as antibody (IgGs) production and neutralizing antibody production. In this case, the negative control group was immunized with PBS. Virus challenge inoculation was performed after nasal infection at a concentration of 2 × 10⁴ pfu/mouse 28 days after initial immunization, and the survival rate and tissue titer were measured (FIG. 8A).

### 1) Antibody induction analysis according to plant vaccine immunization

After separating the serum from the blood of the mice immunized with the prepared plant vaccine, it was confirmed by ELISA whether antigen-specific antibodies were generated in the serum. It was confirmed that sufficient IgG titers appeared after a total of two immunizations, and it was confirmed that both S 1 and S2 specific antibodies, which are subdomains of the S antigen, were generated by this vaccine, and it was confirmed that the group immunized with an immune adjuvant (aluminum hydroxide) at the same time showed a higher IgG titer than the group that was not administered simultaneously (FIG. 8B).

### 2) Analysis of survival rate and tissue titer according to hamster challenge inoculation according to plant vaccine immunization

28 days after initial immunization, the virus was intranasally infected to confirm protection against SARS-CoV-2 infection in transgenic mice whose immunogenicity was confirmed, and the body weight, body temperature and clinical symptoms were observed for 14 days after infection (FIG. 8C). As a result of measuring the change in body weight for 2 weeks after infection, the hamsters immunized with the plant vaccine showed less weight loss compared to the PBS group which was a negative control group. As a result of the survival rate analysis of the plant vaccine immunization group, the 15 and 30 µg immunization groups survived 100%, and the 15 and 30 µg and immune adjuvant-concurrently administered group showed an 80% survival rate. In addition, the survival rate of the control (PBS) immune group was confirmed to be 1 out of 3 survivals (33%) (FIG. 8D). In order to confirm the virus proliferation pattern and histopathological findings in the lung tissue according to the virus challenge inoculation, autopsies were performed on days 3, 5 and 7 after infection, and the virus titer in the autopsied lung tissue was measured. In the plant vaccine immunization group, the inhibition of virus proliferation was confirmed in proportion to the administered dose compared to the control group, and on day 7, no virus was detected in the group administered with 30 µg and the immune adjuvant at the same time (FIG. 8E).

## Claims

1. A recombinant vector for producing a recombinant spike protein of a coronavirus forming a trimer, comprising:
(i) a gene encoding a protein lacking an amino acid sequence from the transmembrane domain to the C-terminus of the spike protein of a coronavirus;
a protein including an amino acid sequence from the N-terminus to sub-domain 2 (SD 2) of the spike protein of a coronavirus; or
a protein lacking an amino acid sequence from the transmembrane domain in subunit 2 to the C-terminus of the spike protein of a coronavirus; and
(ii) a gene encoding a protein of a trimeric motif region of Coronin 1 (mCor1).

2. The recombinant vector of claim 1, wherein the coronavirus is any one selected from the group consisting of SARS-CoV (SARS-Coronavirus), MERS-CoV (MERS-Coronavirus) and SARS-CoV-2 (SARS-Coronavirus-2).

3. The recombinant vector of claim 2, wherein the protein lacking an amino acid sequence from the transmembrane domain to the C-terminus of the spike protein of SARS-CoV-2 comprises the amino acid sequence of SEQ ID NO: 2.

4. The recombinant vector of claim 2, wherein the gene encoding a protein lacking an amino acid sequence from the transmembrane domain to the C-terminus of the spike protein of SARS-CoV-2 comprises the nucleotide sequence of SEQ ID NO: 1.

5. The recombinant vector of claim 2, wherein the protein including an amino acid sequence from the N-terminus to sub-domain 2 (SD 2) of the spike protein of SARS-CoV-2 comprises the amino acid sequence of SEQ ID NO: 4.

6. The recombinant vector of claim 2, wherein the gene encoding a protein including an amino acid sequence from the N-terminus to sub-domain 2 (SD 2) of the spike protein of SARS-CoV-2 comprises the nucleotide sequence of SEQ ID NO: 3.

7. The recombinant vector of claim 1, wherein the protein of a trimeric motif region of Coronin 1 (mCor1) comprises the amino acid sequence of SEQ ID NO: 6.

8. The recombinant vector of claim 1, wherein the gene encoding a protein of a trimeric motif region of Coronin 1 (mCor1) comprises the nucleotide sequence of SEQ ID NO: 5.

9. The recombinant vector of claim 1, wherein the vector is a binary vector.

10. The recombinant vector of claim 1, wherein the recombinant vector further comprises any one promoter selected from the group consisting of a 35S promoter derived from cauliflower mosaic virus, a 19S RNA promoter derived from cauliflower mosaic virus, a Mac promoter, an actin protein promoter and ubiquitin protein promoter of a plant.

11. A transgenic organism which is transformed by the recombinant vector of claim 1.

12. A transformant, in which the transgenic organism of claim 11 which is transformed is a prokaryote or a eukaryote.

13. A method for producing a recombinant spike protein of a coronavirus forming a trimer in a plant, comprising the steps of:
(a) constructing the recombinant vector of claim 1;
(b) preparing a transgenic organism by introducing the recombinant vector into an organism;
(c) culturing the transgenic organism;
(d) infiltrating the culture product into a plant; and
(e) pulverizing the plant to obtain a recombinant spike protein of a coronavirus forming a trimer.

14. A recombinant protein which is prepared according to the method of claim 13.
